# EUROPEAN PATENT APPLICATION

(11) **EP 3 539 512 A1**
(43) Date of publication of application: **18.09.2019**
(21) Application number: 18161139.3
(22) Date of filing: 12.03.2018
(51) Int. Cl.: A61F 2/46

(54) **SHOULDER IMPLANT TRIAL SYSTEM**

(71) Applicant: Arthrex Inc, Naples, FL 34108-1945 (US)
(72) Inventor: CHAUDOT, Audrey, 81675 Munich (DE); RACHLITZ, Ernst, 82205 Gilching (DE)
(74) Representative: Lohr, Jöstingmeier & Partner

(57) **Abstract**

A humeral implant trial embodiment comprising liners (100), spacers (500), and protectors (600) is disclosed. The humeral implant to be used can be accurately identified and exchanged intra OP, if necessity is identified during the trial. By using a combination of the liners, spacers and protectors, the bone anchoring or the cancellous part of the bone normally used for fixing humeral implants is not compromised. The result is an efficient and perfectly placed implant on the head of a recessed bone, avoiding the conventional poor placement and subsequent correction of the implant. It avoids injury to the cancellous bone part and of the surrounding tissue and avoids lengthy, time consuming and strenuous surgery.

## Description

### Field of the invention

The invention relates to the field of surgical tools and, more particularly, to a humeral trial prosthesis used with corrective liners prior to reaming a bed in the humeral bone component for correctly aligning the final implant.

### Description of the related art

During surgical procedures, it is often necessary to approach a specific anatomical location precisely and directly, to avoid unwanted surgical trauma. For example, orthopedic surgery and related trauma care requires bone reaming to allow for implants. These procedures may include hip replacement, knee replacement and shoulder replacement. It is important during surgery to minimize damage and protect surrounding soft tissue.

Shoulder, hip or knee implants are very demanding surgical operations in terms of medical resources, financial resources, and in terms of body invasion. For a hip replacement, it is not unusual to have a whole team of doctors working sometimes in shifts for many hours to set an implant in the right position. It is also not uncommon that these implants are either too long or too short, and therefore cause pain in the joint or are not fixed properly due to too little bone and too much cement used. In some cases, a second surgical operation is needed to repair the failing implant. As such, it is important that the implant fits exactly from the first try.

The prior art document EP 2 959 852 A1 of Jensen, Kirk L. et al. relates to a surgical instrument assembly for use during a surgical procedure to implant a stemless humeral component. This component is to replace the humeral head of a patient's humerus including a surgical punch, configured to punch holes in the patient's humerus to receive a number of legs of the stemless humeral component.

A further prior art document EP 1782765 A1 of Smeltzer Dean E. et al, relates to an implant assembly for shoulder joint replacement. One of the embodiments disclosed provides an assembly of implant components for shoulder joints having a humerus and a glenoid. The assembly includes a plurality of humerus stems, a plurality of heads, and a plurality of cups configured to articulate with the heads. The assembly also includes a plurality of glenoid adaptors for transitional shoulder arthroplasty. In this document, the glenoid fixation component has a first curved coupling surface configured to be mated with a prepared glenoid and an outward facing surface having a boss position for coupling the head to the glenoid fixation component.

After long scientific research, it has been concluded that none of the prior art documents satisfy the need for a simple and flexible trial implant which is easy to use, precise to implement, protects the surrounding soft tissue, is reusable, and is viable more economically.

### Summary of the invention

The problem to be solved by the invention is to provide tools for placing an implant in the optimal position without any surgical aggravations.

Furthermore, there is a need for better, easier, and more precise trial tools without inflicting further injury to the bone more than it is necessary, by removing only the absolute minimum part of the bone and therefore, allowing for a strong healthy placement of the implant.

It is the aim of the present invention to alleviate the above-mentioned problems by preferably using a trial implant which can be connected and disconnected from the bone as many times as necessary. The construct can be manipulated and amended until the result is similar in size and orientation as the original joint.

The trial tool should allow to correctly predict the size, direction, and alignment of a final implant preferably with the help of trial embodiments consisting of liners, off-sets, and protectors.

The solutions to the problem is defined in the independent claims of the application. The dependent claims relate to further improvements of the invention.

The humeral implant to be used can be accurately identified and exchanged intra OP if identified as necessarily during the trial. By using a combination of trial liners and protectors, or trial liners, spacers and protectors the bone anchoring or the cancellous part of the bone normally used for fixing humeral implants is not compromised. These embodiments are used merely as trials prior to the actual implant.

The result is an efficient and accurately placed implant in the bone. This avoids the conventional poor placement and subsequent correction of the implant. It avoids injury to the cancellous bone part and to the surrounding tissue. Furthermore, it avoids lengthy, time consuming and strenuous surgery as well as unnecessary long recovery time.

In a first preferred embodiment, the trial implant system comprises a trial liner, a spacer, and a protector. The trial liner comprises a preferably concave joint surface. The spacer may be arranged below the liner for adjusting the distance of the liner from the bone. The protector protects the bone surface and holds the spacer and the liner onto the bone surface.

The trial liner has a top and a bottom side. Furthermore, it has a concave shaped glenosphere cup on the top side and a central liner snap connector at the bottom side. The trial liner preferably has a fixing nipple to allow the liner to be fixed to a spacer or a protector in a certain angle position.

The spacer has a top side and a bottom side. The spacer further has a central spacer top snap connector at the top side, and a central spacer bottom snap connector at the bottom side. It may also have a restriction pin from the bottom side to fix the spacer in a proposed angular position. The spacer may have a central inner hole which may be circular, to allow the central liner snap connector fit therein. The spacer may further have two or more opposing half shaped grasping channels on the top, on the bottom or on both sides of the spacer. The protector has a protector plate with a top section and a bottom section. The protector plate preferably further comprises a central post extending from the top section through the bottom section of the protector plate, and at least one anchoring pin extending from the bottom section of the protector plate. The protector plate further comprises at least one lateral positioning tooth extending also from the bottom section of the protector plate. The at least one anchoring pin may protrude into a hole of the bone and provide a higher pull-out force of the protector. The at least one positioning tooth serves to fix the protector and therefore the spacer and liner onto the bone. The protector may also be formed with at least one lateral positioning tooth without any anchoring pins extending from the bottom section of the protector plate. The central post may further comprise a snap connector. The central post may be placed centrally but may also be offset, depending on the anatomical circumstances of the bone.

When attached to a bone, the central post extends into a hole of the bone. The central post may have a diameter in a range of 8 mm to 15 mm, preferably between 12 mm and 14 mm and most preferably of 13,2 mm. The at least one anchoring pin extending from the bottom section of the protector plate may be very thin and short compared to the central post. The at least one anchoring pin may have a radius of less than 20 % of the central post and a height less than the central post. When more than one anchoring pin is needed, these may be placed opposite to each other with the central post in between. In certain circumstances the pins may also be placed just next to each other. The at least one anchoring pin helps to hinder the protector plate from rotating during the trial operation.

The at least one lateral positioning tooth extending from the bottom section of the protector plate is shorter and thinner in size than the central post. It is as well shorter than the anchoring pins. It may have only one ridge or a plurality of ridges to hold the protector in the bone, such that they only minimally invade the bone when in use. The at least one lateral positioning tooth is used for temporarily securing the protector to the recessed part of a bone. The central post of the protector may have a through-hole forming a central post snap connector. The first part of the through-hole may start from the top section and may have at least one large diameter which may be threaded. This is to allow fixation of a handle impactor/retractor to the protector.

In this embodiment, the central liner snap connector fits into the central spacer top snap connector of the spacer. The central spacer bottom snap connector fits into the central post snap connector of the protector.

In a further preferred embodiment, the rest of the trough-hole may have a smaller diameter. The remaining internal wall of the central post may have undulations and ridges at preferably regular intervals. The intervals of the ridges may be from 1- 5 mm. The ridges do not necessarily have all the same size. For instance, the first ridge may be smaller in size than the last ridge or vice versa.

In another embodiment, the central liner snap connector may be fixed directly into the central post snap connector of the protector without any spacer in between.

In a further preferred embodiment, the protector also has several angular holes on the top section. These may be through-holes penetrating the entire plate from the top section to the bottom section or just may be holes in the top section of the protector plate.

These holes are aligned such that a restriction pin from a spacer or a fixing nipple from a liner is able to penetrate said holes. Depending on the system needed, either with or without a spacer the restriction pin of the spacer or the fixing nipples of the liner can protrude through one of these holes to align said spacer or liner in the right angular orientation. The angular holes may be placed such that the angle of alignment can be oriented in predefined steps having a size between 5 to 20 and preferably 10 degrees.

The protector may be made of plastic, hard plastic, a resin, or a metal which may withstand the pressing or ramming force applied to it when inserted into the bone. The resin, plastic, or metal may have a different color than the rest of the device to be easily distinguishable.

In a further embodiment, a trial liner may be attached directly to the protector. The liner may have spread feet extending from the center base of the bottom side of the liner. The spread feet may have at least one ridge or undulation on their exterior. The external ridges may match the inner ridges of the central post of the protector or those of a spacer allowing the liner to be held in its place when inserted and fitted in the central post snap connector of the protector. The spread feet are preferably designed to be compressible or flexible when inserted in the central post snap connector of the protector or when inserted into the central spacer top snap connector of the spacer to engage a tight fit. Preferably, there may be at least two spread feet close to each other with a small gap of a few millimeters between them. This gap may be between 0.5 - 5 mm. The spread feet may extend parallel to each other in the distal direction and may be flexible enough to be compressed towards each other. The spread feet may not extend further than the end of the central post of the protector module. The flexible spread feet may be compressed slightly to allow their ridges to pass the ridges of the protector or those of a spacer until the liner cannot be pushed in any further. The liner may remain in place due to the friction force between the flexible spread feet and its ridges connected to the inner ridges of the protector or those of the spacer.

Preferably, there are different kinds of liners such as standard liners, standard constrained liners, corrective liners, and/or corrective constrained liners.

The standard liners preferably are geometrically uniform. This means the wall of the glenosphere has the same height in all directions, and the glenosphere is not inclined. The standard constrained liner is also a geometrically uniform liner, however, it may have a higher raised cup wall. There may be standard constrained liners which may have a 2 mm or 3 mm or even 4 mm higher cup wall to better receive the matching ball head of the joint.

There may be corrective liners which have an inclined glenosphere by having a higher raised cup wall on one side of the liner. Finally, there may be corrective constrained liners which have both an inclined glenosphere due to a higher raised cup wall on one side and a higher cup wall in general.

Preferably, the liners are adjusted by rotating and pressing in until it sits accurately or flush with the protector or the spacer. The fixing nipple of the liner may enter the angular hole of the protector or the fixing nipple hole of the spacer to place the liner at the correct lateral angle. The nipple also hinders the liner from rotating in place.

Once the liner is in place, the surgeon may determine if the entire system, as it stands, would fit precisely to the rest of the human shoulder. If this is not the case and the directly placed liner on a protector is too low, a spacer may be inserted between the protector and the liner.

The spacer is preferably disc-shaped, but it could also have a different irregular form. Normally, it has a similar form and radial size as the liner. The spacer lifts the liner by a predetermined distance from the protector. The spacer is preferably made of plastic or resin, or even of metal and may have a different color than the liner or the protector. Its exterior radial surface is preferably formed with ridges so that it does not slip between the surgeon's fingers when it needs to be adjusted or removed from between the liner and protector.

The spacer's central inner hole may allow the spread feet of the liner to pass through. The central inner hole extends into an inner tube which preferably has the same size as the inner tube of the protector. The first part of the inner tube, starting from the proximal end or the top part of the spacer preferably has at least one larger diameter which may be threaded.

In this preferred embodiment the larger diameter may be used to allow fixation of a handle impactor/retractor to the protector and to allow the spacer to be extracted or placed in the right position. The remaining of the inner tube may have a smaller diameter. The inner tube may have an inner wall with ridges. The wall may comprise undulations/bulges and ridges/valleys, which may be at regular intervals. The intervals of each bulge or valley in the wall ridges can be from 1 - 5 mm. The ridges do not necessarily have the same size. For instance, the first may be smaller in size than the last ridge or vice versa. The inner tube may end in a pair of flexible spread feet similar in size as the flexible spread feet of the liners. When a spacer is needed in a trial, the liner's flexible spread feet may be inserted in the inner tube of the spacer, wherein the flexible spread feet of the spacer are inserted in the through-hole of the protector. Preferably, they all are maintained in place due to the pressure force exerted upon the flexible spread feet.

An alternative way to extract the liner from the spacer or the spacer from the protector could be done with the help of the lateral protector or lateral spacer grasping channels which start at the edge of the spacer and may extend all the way to the central inner hole. The lateral grasping channels may have a flat base throughout or they may be wedged. The wedge shaped grasping channels are preferably larger at the edge of the spacer and continuously smaller towards the center of the spacer.

These grasping channels allow a forceps, a grasper, a pointing or a flat sharp tool such as an osteotome, to be inserted between the spacer and the liner or between the spacer and the protector in order to gently dislocate them from each other.

The liners, the spacers, and the protectors may have different diameters. As such there may be different sizes of liners fitted to different sizes of spacers and protectors. Preferably, however the liners fit to matching spacers and liners. These are chosen depending on the size of the recessed bone head of the patient.

A color, a number, a letter, a sign, a symbol, or any other form of identification system can be provided for the three modules, such that an inadvertent mistake in using a liner with a wrong spacer or protector is prohibited. The operator may easily identify the colors, numbers, letters, signs, or symbols and may choose the right compatible fitting devices. This raises confidence in the tools used and shortens the time for lengthy surgical operation by not having to try out first if the pieces used are compatible with each other.

One of the advantages of this system is that the bone head itself is only minimally invaded by the three anchoring pins, by a thin central post and the lateral positioning teeth. Most of the cancellous or spongy bone remains intact after the procedure and can be used for a proper fixation of a subsequent implant.

Furthermore, a method for alignment of a final implant with the help of the trials consisting of liners, spacers, and protectors is described.

In a pre-operative step the planning and measuring of the anatomic neck and angle of the bone is performed.

In an operative step the operator resects the top part of a bone joint based on the measurement results and according to a state of the art method of resection. A plastic or metal template may be placed on the resected surface of the recessed head to assess the size of the protector required. The template may be exchanged until the right size is found. The template may have a handle extending in an upper lateral direction, a central through-hole and a few pins extending in the lower direction. The handle allows the operator to move the template to an exact location on the resected surface. Once the optimal place is reached, the operator presses the template into the cancellous bone. The pins allow the template to remain secure on top of the resected surface. Using a drill bit or a reamer, a pre-marked and defined short hole is drilled via the through-hole into the spongy bone. Subsequently the template is removed and a protector of the right size as described above is pressed or reamed in its place in the pre-marked drilled holes. The protector is maintained in its position due to its at least one anchoring pin and the at least one lateral positioning tooth. The spacer may be placed on the protector, and the trial liner may be mounted on top of the spacer.

In a further step, the operator selects the trial liner to be used from a set of liners. If necessary, a spacer may also be chosen from a set of spacers. The liner may be pushed through the central inner hole of the spacer until the base of the spread feet is securely fixed to the spacer. In a second step, the liner connected to the spacer may then be placed on the protector and pushed towards the protector such that the spread feet of the spacer enter through the protector plate inner hole of the protector.

Alternatively the spacer is first placed and secured in the protector and subsequently the liner is place and secured in the spacer.

In a further alternative the liner may be placed directly on the protector and the spread feet of the liner may be pushed through the protector plate inner hole of the protector directly without the use of a spacer.

Before a corrective liner is pressed completely into the final position, the spacer and the liner are angularly adjusted. This step is superfluous with a normal or a constrained liner since the angle of rotation of the liner does not change the geometry of the system or the angle of the liner. However, this is necessary when a corrective or a corrective constrained liner is used.

As a further step the operator assesses if the system composed of the three or only two components satisfies the requirements of the joint to be replaced. If the liner appears to be too low (too short) hence, there is too much slack in the joint, the surgeon may remove the liner by gripping it laterally with the hand. If the liner or the spacer prove to be too difficult to extract, a grabbing instrument such as a forceps or grasper is used. Alternatively, a pointing device or a flat sharp tool such as an osteotome is inserted in the lateral grasping channels to help pop out the liner or the spacer from the protector. In another preferred method the operator may use the impactor/extractor handle to have enough traction power to pull the liner or the spacer out of the protector. A thinner or a thicker spacer is then used for a new trial. During this process, the flexible spread feet of the liner preferably compress towards each other and allow the liner and the spacer to be removed without displacing the protector from its fixed position on the bone. The same process may be necessary if the angle of orientation needs to be adjusted. The liner is carefully extracted a couple of millimeters, until the fixing nipple of the liner and/or the spacer nipple of the spacer are released and the liner can be adjusted in an angular rotation. The liner may then be re-inserted in the spacer or the protector at an adjusted corrected angle.

When the operator is satisfied with the height and the angular orientation of the system, the liner and the spacer are removed and set aside for later use and identification. The protector is subsequently removed with an impactor/extractor tool. The operator can gently pull the handle and extract the protector without damaging the bone or the protector.

Throughout the entire procedure, the protector remains fixed to the bone allowing for a precise placement and measurement of the trial joint. The liner, spacer and protector together correspond to a precise defined implant size. As such, the operator needs only to select the correspondingly sized implant and proceed with implanting it in the prepared place exactly and accurately.

The instruments, systems and methods may be applied to a variety of fields, for example, sports medicine, implant surgery, trauma, etc.

### Description of Drawings

The invention will now be described, without limitation of the general inventive concept, by way of example on embodiments with reference to the drawings.
Figure 1 shows a trial system comprising a protector, a spacer and a corrective liner fitted together on a recessed bone head.
Figure 1a shows a view from the front of a standard liner.
Figure 1b shows a view from the side (90°) of a standard liner.
Figure 1c shows a view from the back of a standard liner.
Figure 1d shows a view from the bottom of a standard liner.
Figure 1e shows a view from the top of a standard liner.
Figure 2a shows a view from the front of a standard constrained liner.
Figure 2b shows a view from the side (90°) of a standard constrained liner.
Figure 2c shows a view from the back of a standard constrained liner.
Figure 2d shows a view from the bottom of a standard constrained liner.
Figure 2e shows a view from the top of a standard constrained liner.
Figure 3a shows a view from the front of a corrective liner.
Figure 3b shows a view from the side (90°) of a corrective liner.
Figure 3c shows a view from the back of a corrective liner.
Figure 3d shows a view from the bottom of a corrective liner.
Figure 3e shows a view from the top of a corrective liner.
Figure 4a shows a view from the front of a corrective constrained liner.
Figure 4b shows a view from the side (90°) of a corrective constrained liner.
Figure 4c shows a view from the back of a corrective constrained liner.
Figure 4d shows a view from the bottom of a corrective constrained liner.
Figure 4e shows a view from the top of a corrective constrained liner.
Figures 5a and 5d show a larger and a smaller spacer from the bottom side.
Figures 5b and 5e show a larger and a smaller spacer from a front lateral side.
Figures 5c and 5f show a larger and a smaller spacer from the top side.
Figures 6a and 6d show a larger and a smaller protector from the bottom side.
Figures 6b and 6e show a larger and a smaller protector from a front lateral side.
Figures 6c and 6f show a larger and a smaller protector from the top side.
Figure 7 shows a liner snap connector and a spacer snap connector fixed together in the protector central post snap connector area.
Figure 8 shows a sectional lateral view along the vertical axis of a fitted embodiment comprising a standard liner, a spacer, and a trial element.
Figure 9 shows a sectional lateral view along the vertical axis of a fitted embodiment comprising a corrective liner, a spacer, and a trial element.
Figure 10a shows a lateral view of a protector plate fixed on bone and the fixing end of an impactor/extractor tool.
Figure 10b shows a lateral view of an impactor/extractor tool.
Figure 10c shows a lateral view of the fixing end of an impactor/extractor tool.
Figure 11a shows a liner having a square lock and gripping channels
Figure 11b shows a lateral view of the liner with the square lock
Figure 11c shows the top of the liner
Figure 11d shows a liner having a rectangular lock and gripping channels
Figure 11e shows a lateral view of the liner with the rectangular lock
Figure 11f shows the top of the liner
Figure 12a shows a spacer having a square lock and gripping channels
Figure 12b shows a lateral view of the spacer with the square lock
Figure 12c shows the top of the liner and the square lock space
Figure 12d shows a spacer having a rectangular lock and gripping channels
Figure 12e shows a lateral view of the spacer with the rectangular lock
Figure 12f shows the top of the liner and the rectangular lock space
Figure 13a shows an enlarged cut through lateral view of a spacer
Figure 13b shows an enlarged top view of a spacer and the cut through plane
Figure 14a shows the bottom part of a protector
Figure 14b shows a lateral view of the protector
Figure 14c shows the top part of a protector having a square lock
Figure 14d shows the bottom part of a protector
Figure 14e shows a lateral view of the protector
Figure 14f shows the top part of a protector having a square lock

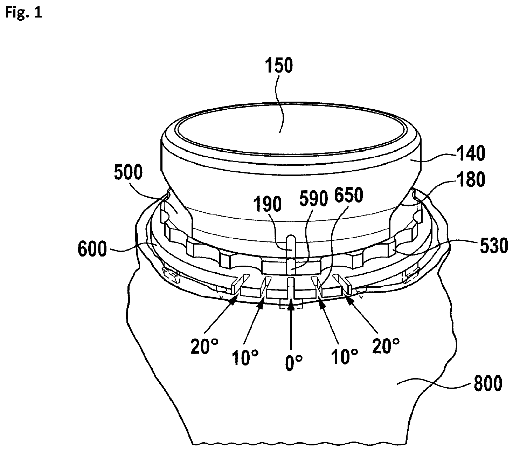

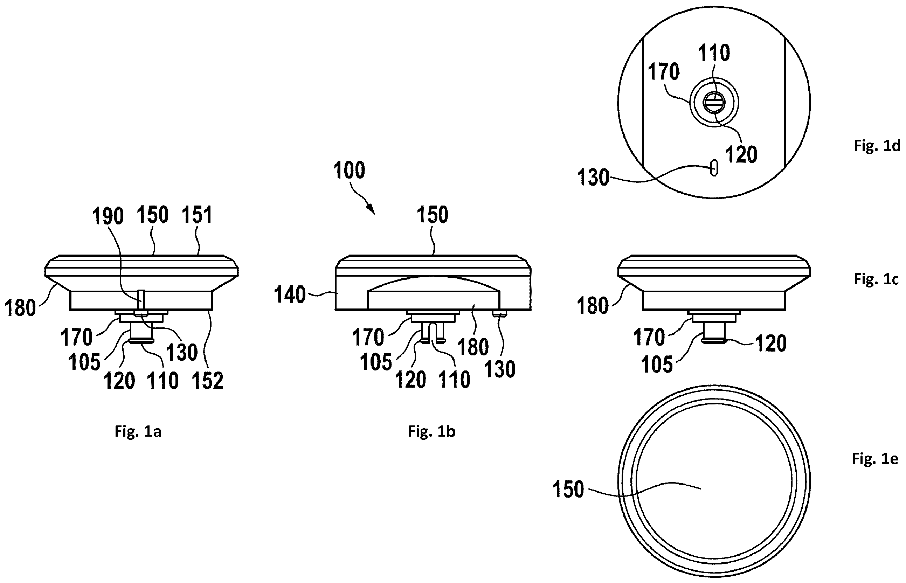

Figure 1 depicts an example of a complete trial system in use. Here, the standard liner 100, the glenosphere cup 150, the angle direction lines 190, the spacer 500 and the protector plate 600 can be easily identified. The angular markings 650 on the protector plate 600 are specified as 10° angular steps. By rotating the spacer 500 to the next angular marking 650 the liner 100 also moves, therefore changing the geometry of the joint in 10° steps.

Figures 1a-1e show views along all three axes of a standard liner 100. The standard liner 100 has a top side 151 connected by a cup wall 140 to a bottom side 152. Figure 1a shows a view from the front. Figure 1b shows a lateral view (turned by 90°. Figure 1c shows a view from the back. Figure 1d shows a bottom side view and Figure 1e shows a top side view. The liner has a concave glenosphere cup 150 on the top side 151 suitable for receiving a ball bone joint. The glenosphere cup 150 is formed within the cup wall 140. Said cup wall 140 has lateral finger recesses 180 on the opposite sides to permit a user to securely grab hold onto the liner without the liner slipping from the user's fingers during use. Also on the lateral wall, there is at least a mark or an angle direction line 190 which allows the liner 100 to be rotated angularly around a vertical central axis. Extending from the center of the bottom side 152 of the liner 100 is a central liner snap connector 105. In a preferred embodiment, the central liner snap connector 105 may have at least two spread feet 110. Laterally thereof may be at least one fixing lateral nipple 130. The spread feet 110 may have a spread feet base 170 which may be fixed connected to the bottom central area of the liner 100.

In another embodiment, the spread feet 110 may have at least one ridge or undulation 120 on their exterior, which is preferably at the end point, away from the liner, on the spread feet 110. Preferably the spread feet 110 may have at least two spread feet close to each other with a small gap of a few millimeters in between. This gap can be between 0.5 - 5 mm. The spread feet 110 may extend parallel to each other and are facing away from the liner's bottom side 152. The spread feet 110 may be flexible enough to be compressed towards each other many times without breaking.

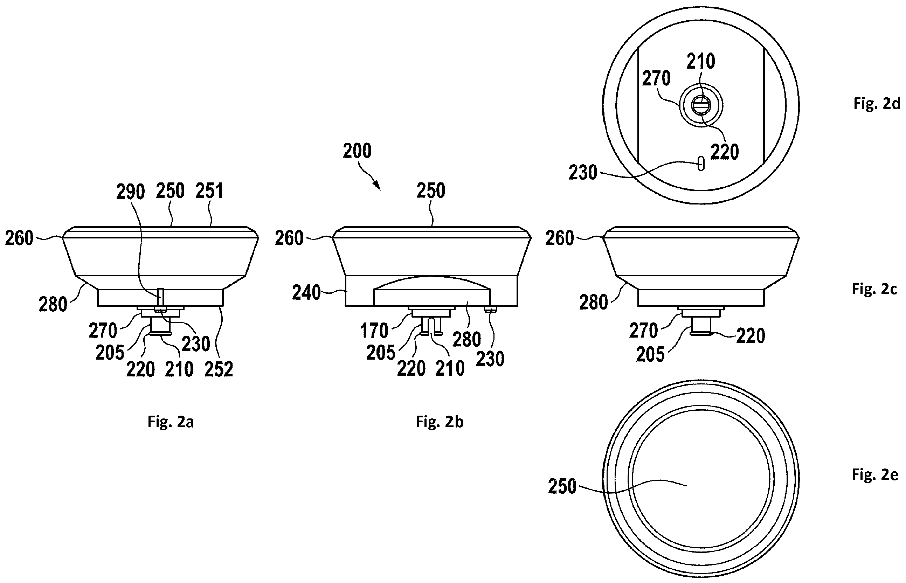

Figures 2a-2f shows a view along all three axes of a standard constrained liner 200. The standard constrained liner 200 has a top side 251 connected by a cup wall 240 to a bottom side 252. Figure 2a shows a view from the front. Figure 2b shows a lateral view rotated by 90°. Figure 2c shows a view from the back. Figure 2d shows a bottom side view and Figure 2e shows a top side view. The liner 200 has a glenosphere cup 250 at the top side 251 and lateral finger recesses 280 on the side. A central liner snap connector 205 extends centrally from the bottom side 252.

In a preferred embodiment, the cup wall 240 extends away from the top side 251 of the liner further than the cup wall 240 of the standard liner 100. The difference in the height of the wall compared to a standard liner 100 may be between 0.5 mm and 10 mm. This extension of the cup wall 240, identified as the constrained wall 260, allows a bone ball joint to be better supported, prohibiting it from slipping out of the glenosphere cup 250. The use of a constrained liner 200 is necessary when the humeral joint is not pulled together tight enough by the ligaments, therefore allowing the ball bone joint to dislocate. Using a constrained liner 200 gives the patient a more secure support. But at the same time, it may reduce the degree of movement of the arm in the shoulder.

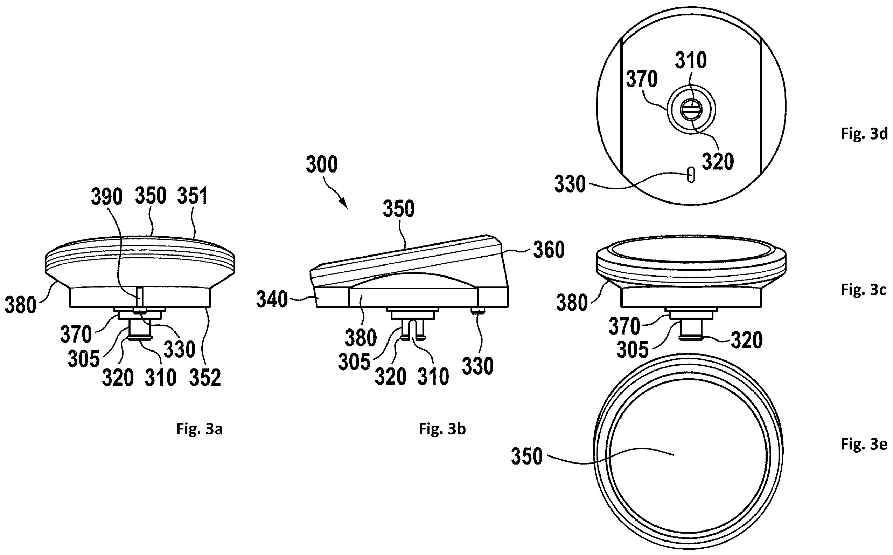

Figures 3a-3f shows a view along all three axes of a corrective liner 300. The corrective liner 300 has a top side 351 connected by a cup wall 340 to a bottom side 352. Figure 3a shows a view from the front. Figure 3b shows a lateral view rotated by 90°. Figure 3c shows a view from the back. Figure 3d shows a bottom side view and Figure 3e shows a top side view. The liner has a glenosphere cup 350 at the top side and lateral finger recesses 380 on the side. A central liner snap connector 305 extends centrally from the bottom side 352.

Whereas in a constrained liner 200 the cup constrained wall 260 has the same height at all sides, in a corrective liner 300 the cup wall 340 is higher on one side. The raise only on one side of the cup wall 340, identified as the corrective wall 360 allows the shoulder more degree of freedom in one direction than in another. Using a corrective liner 300 may also depend on the anatomical shape of a patient's shoulder joint. Hence, using a corrective liner 300 may save the patient the pain of another bone recess at an angle.

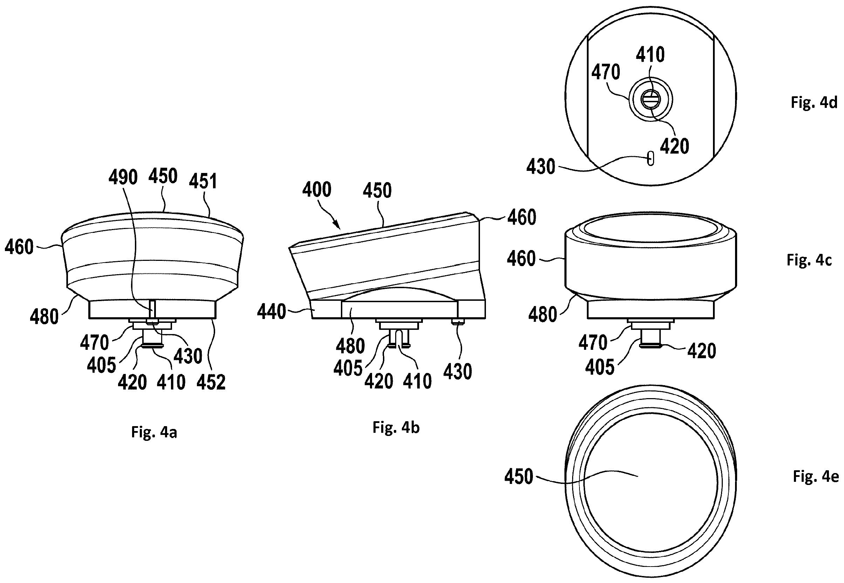

Figures 4a-4f shows a view along all three axes of a corrective constrained liner 400. The corrective constrained liner 400 has a top side 451 connected by a cup wall 440 to a bottom side 452. Figure 4a shows a view from the front. Figure 4b shows a lateral view rotated by 90°. Figure 4c shows a view from the back. Figure 4d shows a bottom side view and Figure 4e shows a top side view. The liner 400 has a glenosphere cup 450 at the top side and lateral finger recesses 480 on the side. A central liner snap connector 405 extends centrally from the bottom side.

In this preferred embodiment, the cup wall 440 is raised higher than the cup wall of the corrective liner 300. This further height raises of the cup wall 440, identified as the corrective constrain wall 460, allows the glenosphere cup to surround a ball bone more thoroughly, therefore providing more security against dislocation.

The angle direction lines 190, 290, 390, 490 indicate the degree of angular rotation of the liners.

The trial parts may be molded, extruded or 3D printed out of a material such as a plastic, a resin or a rubber, or any other material being biologically compatible with the human body. The color may for example be gray, blue, green or red. They may also have any other color than the rest of the trial parts. The trial parts may be provided in different sizes. An operator could choose the appropriate size of the liner depending on the anatomical necessities.

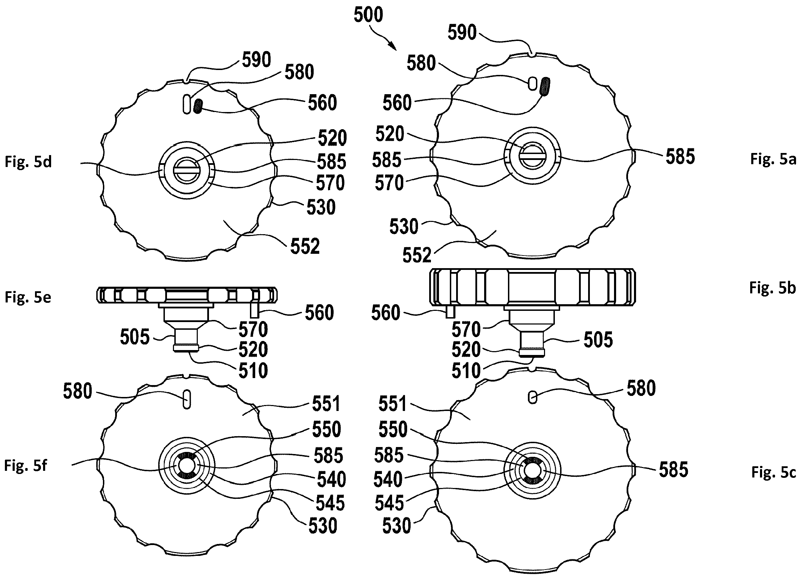

Figures 5a -5f show a larger version of the spacer 500 from different angles, whereas Figures 5d - 5f show the smaller version of the spacer 500. The spacer 500 has a top side 551 and a bottom side 552. Figures 5a and 5d show a bottom side view of the spacer 500. Figures 5b and 5e show a lateral or front view of the spacer and Figures 5c and 5f show a top side view of the spacer 500. This embodiment has a flat top side 551 and lateral finger ridges 530 on the side suitable for the user to grab the spacer and rotate it when necessary without slipping out of his hand. A central liner snap connector 505 extends centrally from the bottom side 552. Wherein the central liner snap connector 505 is identical in size to any of the central liner snap connectors 105, 205, 305, 405, the rest of the spacer 500 may have different heights depending on the height of the total implant needed. The assessment of the height of the spacer is done by the operator in function of the anatomical necessities in each individual procedure. The trial spacer 500 may have any height from 0.5 mm to 10 mm. More preferably, the spacer 500 may be between 3 mm to 6 mm in height. The spacer 500 preferably has a central spacer inner hole snap connector 540 at the top extending through the spacer. A central spacer snap connector 505 extends centrally from the bottom side.

In a preferred embodiment, the central spacer snap connector 505 may have a spread feet base 570 extending from the bottom side 552. The spacer may have at least two spread feet extending centrally further away from the spread feet base 570. At the top side 551 of the spacer, there may be a central spacer inner hole snap connector 540 which may be circular in shape to allow the spread feet of a liner to pass through. The top part of the central spacer inner hole snap connector 540 may be larger in diameter than the rest of the central spacer inner hole snap connector 540. At least a part of the larger top part of the central spacer inner hole snap connector 540 may be threaded for a handle impactor/retractor or removal tool 700 to be fixed to the protector plate and allow the spacer to be extracted or placed in the right position by a user.

In a preferred embodiment, the central spacer inner hole snap connector 540 extends into an inner tube 550 which may have an inner wall with at least one spacer inner ridge 545. The at least one wall inner ridge 545 may be at regular intervals. The intervals between the inner ridges 545 may be from 1 - 5 mm. The inner tube 550 may be closed before the spread feet 510 start. In a preferred embodiment however, there may also be a penetration in the bottom part of the spread feet base 570 for fluids to sicker through. Alternatively the penetration is a central spacer snap connector transverse channel 595 crossing the snap connector through sideways. Preferably, the spacer 500 has an angle indicator line 590 which is easily identifiable between the lateral finger ridges 530. The angle indicator line 590 indicates the radial angle of the liners 100, 200, 300, 400 used. The radial angle is always 0° for a liner having walls at the same height, like the standard liner 100 or standard constrained liner 200. No matter how the spacer 500 or the liners 100, 200 in these examples are rotated, there is no difference in the angle of the trial liners. If the liner is a corrective liner 300 or a corrective constrained liner 400, the radial angle changes the geometry of the trial. Preferably, the spacer 500 has a spacer nipple 560 which allows the spacer 500 to be accurately placed in a specific position over a protector plate 600 which may be already fixed to the bone. The spacer 500 also may have a spacer fixing nipple hole 580 which allows the lateral fixing nipples 130, 230, 330, 430 from the liner 100, 200, 300, 400 to be placed accurately in the fixing nipple hole 580 to allow for a specific direction placement of the liners over the spacer 500. The spacer fixing nipple hole 580 of a shallow spacer may be of a different size or different location on the spacer to prevent that wrong liners are placed thereon. Too large liners are not compatible to be placed on too small trial spacers.

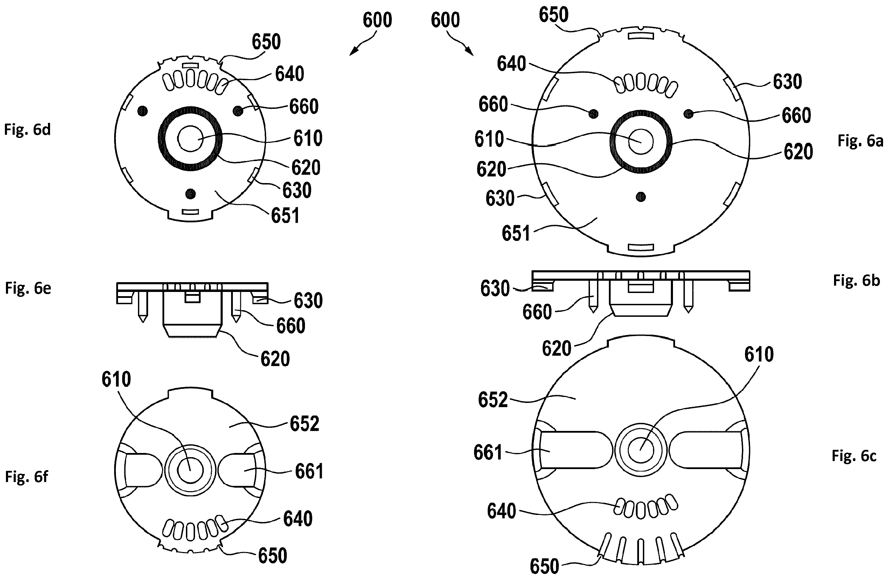

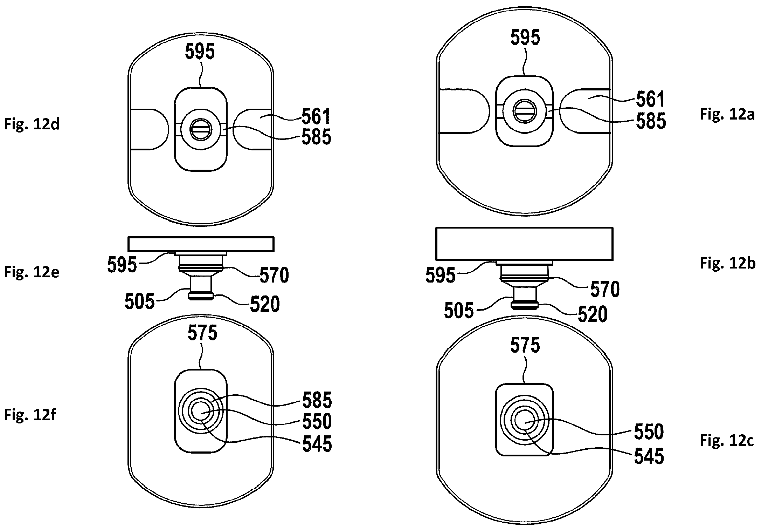

In another preferred embodiment shown in figures 6a-6f or 12a and 12d the extraction of the liners 100, 200, 300, 400 from the spacer 500 or the extraction of the spacer 500 from the protector 600 could also be done with the help of the lateral liner grasping channels 161, 261, 361, 461 or lateral spacer grasping channels 561 or lateral protector plate grasping channels 661 respectively. These channels 161, 261, 361, 461, 561, 661 start at the edge of the liners 100, 200, 300, 400 or spacer 500 or that of the protector 600 and may extend all the way to the liners feet base 170 or spacer's inner tube 550 or the protector's plate inner hole 610. The lateral grasping channels 561 and 661 may have a flat base throughout. These grasping channel may be constant in size towards the center of the spacer and have a width of 1mm to 20mm, preferably 5mm-15mm and most preferably 8mm-12mm.

In a preferred alternative the base of these lateral grasping channels 161, 261, 361, 461, 561 and 661 may be slightly curved or elliptical and wedge shaped. These grasping channels are preferably larger at the edge of the liners 100, 200, 300, 400 or of the spacer 500 or of the protector 600 and continuously shallower towards the center of the liners or the center of the spacer or that of the protector. The diameter of the wedge shaped grasping channel at the edge of the liner or spacer or that of the protector may be from 1mm to 20mm, preferably 5mm-15mm and most preferably 8mm-12mm.

These grasping channels 161, 261, 361, 461, 561, 661 allow a forceps, a grasper, a pointing or a flat sharp tool such as an osteotome, to be inserted between the spacer 500 and a liner 100, 200, 300, 400 or between the spacer 500 and the protector 600 in order to gently dislocate them from each other. This is done either by pressing the forceps together in the wedged shaped grasping channels 161, 261, 361, 461, 561, 661 respectively, or by inserting the forceps in the flat base shaped grasping channels 561 or 661 and pulling the spacer 500 out of the protector 600, or a liner 100, 200, 300, 400 out of the spacer 500.

In another preferred alternative the forceps or graspers have wedged or conic shaped ends. These wedged or conic shaped forceps or graspers are inserted in either the wedged grasping channel 161, 261, 361, 461, 561, 661 or the flat base grasping channels 161, 261, 361, 461, 561, 661 and by pressing them together it would dislocate the spacer 500 from the protector 600 or a liner 100, 200, 300, 400 from the spacer 500.

In a further preferred alternative a pointing, a wedged, a conic or a flat sharp tool such as an osteotome, or a combination thereof may be inserted in either the wedged grasping channel 161, 261, 361, 461, 561, 661 or the flat base grasping channels 161, 261, 361, 461, 561, 661 respectively to dislocate the spacer 500 from the protector 600 or to dislocate a liner 100, 200, 300, 400 from the spacer 500. This dislocation could be achieved by carefully forcing the tools in the channels through a continuous force or with the help of a hammer or other suitable hitting objects by slightly knocking the pointing, the wedged, the conic or the flat sharp tool such as an osteotome, in the channels.

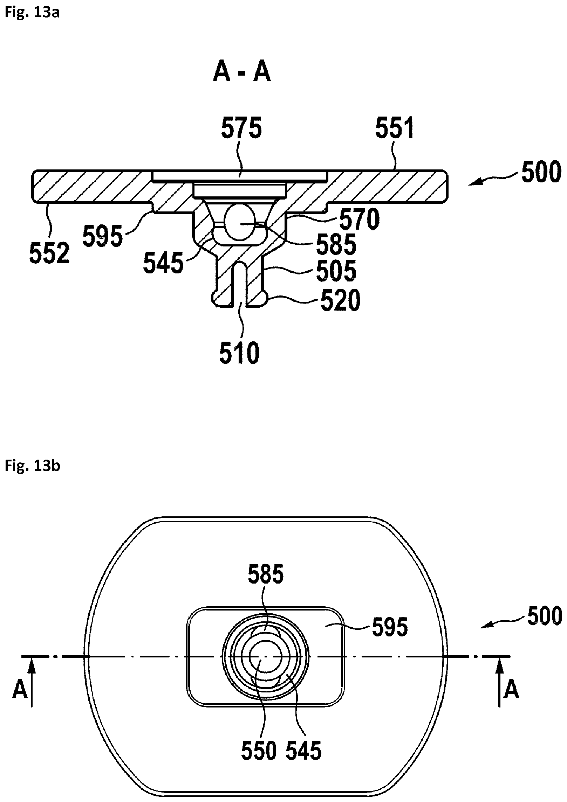

In these preferred embodiments the spacer has only two circular opposing edges connected by two straight sides. This is shown figure 13b. These two straight sides accommodate the liners lateral finger recesses 180, 280, 380, 480 in order to be easily turned and removed.

Preferably the spacer may be molded, extruded or 3D printed out of a material such as a plastic, a resin or a rubber, or any other material being biologically compatible with the human body. The color may for example be gray, blue, green, or red. They may also have any other color which is different than the colors of the rest of the trial parts. The trial parts may be provided in different sizes. An operator could choose the appropriate size of the spacer depending on the anatomical necessities.

Figures 6a - 6f show a large version of the protector plate 600 from all three axes, whereas Figures 6d - 6f show a smaller version of the protector plate 600. The protector plate 600 further has a top section 651 and a bottom section 652. Figures 6a and 6e show the bottom portion of the protector plate 600. Figures 6b and 6e show the lateral or front view of the protector plate 600, and Figures 6c and 6f show the top portion view of the protector plate 600. This embodiment further has a central post 620 extending centrally from the top section 651 through the bottom section 652 of the protector plate 600 and at least one anchoring pin 660 extending from the bottom section 652 of the protector plate 600. At least one lateral positioning tooth 630 may extend from the bottom section 652 of the protector plate 600. The preferred radial size of the protector plate is chosen according to the anatomical need of the patient. In a preferred alternative the protector plate 600 may lack anchoring pins or positioning teeth. The protector may be fixed in the bone only by the central post 620.

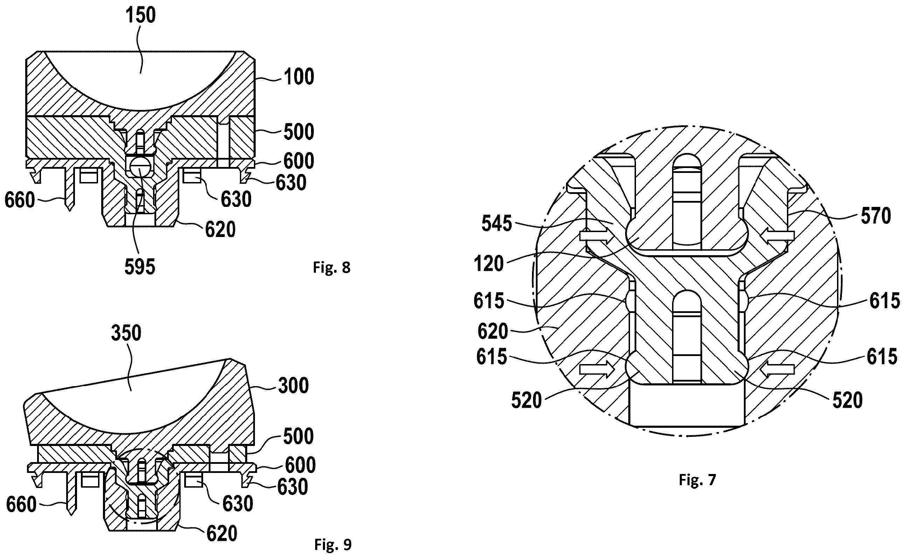

Preferably, on the top section 651 protector plate there is a protector plate inner hole 610. A central spacer snap connector 505 extends centrally through the protector plate inner hole 610 and the central post 620. The size and shape of the central spacer snap connector 505 may be identical to the central liner snap connectors 105, 205, 305, 405 in any operation procedure. The central post 620 may have protector plate inner ridges 615 as can be best seen in figure 7.

In a preferred embodiment, the spread feet ridges 120, 220, 320, 420, 520 and the spread feet bases 170, 270, 370, 470, 570 of the liners 100, 200, 300, 400 and spacer 500 may match the inner protector plate ridges 615 of the central post 620 of the protector plate 600 respectively. This allows the liners 100, 200, 300, 400, or the spacer 500 to be held in place once inserted and fitted in the protector plate 600. In this preferred embodiment, the central liner snap connectors 105, 205, 305, 405 or the central spacer snap connector 505 may have spread feet 110, 210, 310, 410, or 510 preferable designed to be compressible when inserted in the protector plate central post 620 to engage in a tight fit. The spread feet may be shorter, equal or extend longitudinally further than the end of the central post 620 of the protector plate 600.

The anchoring pins 660 are also located on the bottom section 652. In a preferred embodiment, these may surround the central post 620. Preferably, there is at least one anchoring pin 660 on every protector. Laterally, on the perimeter of the protector plate 600 extending away from the bottom section 652 of the protector plate is the at least one lateral positioning tooth 630 used for positioning the protector plate 600 in place during a surgical procedure. The shape of the at least one lateral positioning tooth 630, when seen from a sectional lateral view, may resemble the form of an arrow or half arrow or hook, to easily penetrate the bone 800 and to be difficult to dislocate when pulled out from the bone 800. This arrangement secures firmly the protector plate 600 to the bone 800. The protector plate 600 may have angular holes 640 for receiving the spacer nipple 560 of a spacer 500 or the lateral fixing nipple of a liner. The angular markings 650 on the side of the protector plate 600 allow the spacer 500 and the liners 100, 200, 300, 400 to have a starting and ending point when an angular adjustment is necessary. The liners 100, 200, 300, 400 have angle direction lines 190, 290, 390, 490 on their sides which align with one of the angular markings 650 of the protector plate and the angle indicator 590 of the spacer to give an accurate angular position.

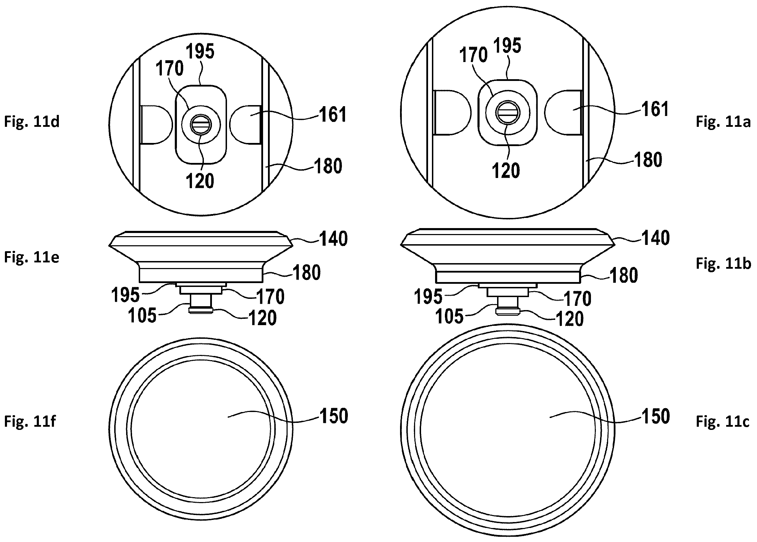

In an alternative embodiment in Figure 11a-11f the liners 100, 200, 300, 400 may lack the lateral fixing nipples 130, 230, 330, 430 and the spacer 500 may lack the respective spacer fixing nipple hole 580. Instead the liners 100, 200, 300, 400 and the spacer 500 may be set in a fixed place through the rectangular rotation lock 195, 295, 395, 495 and 595. The rectangular liner rotation lock 195, 295, 395, 495 and the rectangular spacer rotation lock 595 are a small, either rectangular or square displacement, situated between the bottom side 152, 252, 352, 452, 552 and the spread feet base 170, 270, 370, 470 and 570. The spacer 500 may also have a rectangular rotation spacer lock space 575 to allow the rectangular liner rotation lock 195, 295, 395, 495 to be placed therein. This can be seen in figures 12a-12c. A cut view of a spacer with a rectangular rotation lock can be seen in figures 13a and 13b

Depending of the shape of the rectangular rotation spacer lock space 575 and that of the rectangular liner rotation lock 195, 295, 395, 495 which may be either rectangular or square, these may be places in either two positions (0° or 180°) for a rectangular lock or four (0°, 90°, 180°, 270°) positions for a square lock.

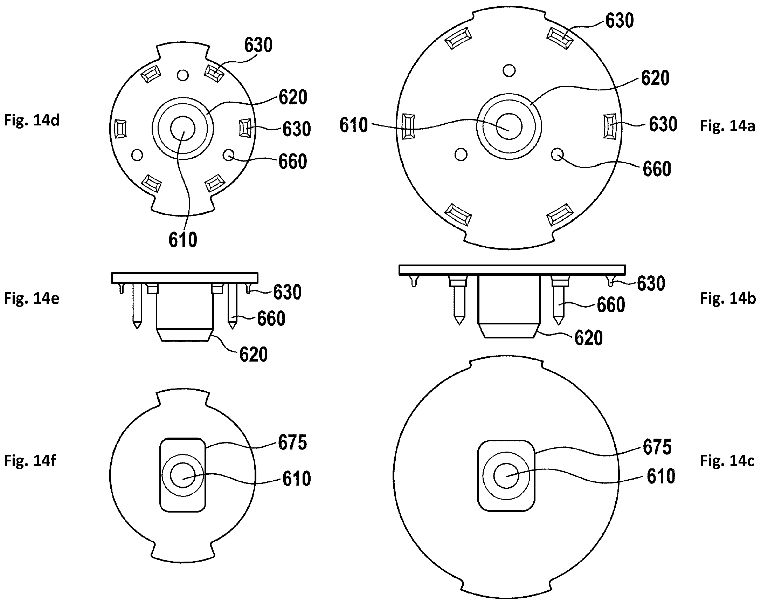

In this alternative embodiment the protector plate may also lack the angular holes 640 and have instead a rectangular rotation protector plate lock space 675 to enable either the liners 100, 200, 300, 400 or the spacer 500 to be set in the rectangular rotation plate lock space 675 as this can be seen in figures 14a-14f.

All the above described protector plates 600 may lack any anchoring pins or positioning teeth and may be fixed in the bone only by the central post 620.

Figure 8 shows a sectional lateral view of a complete trial system comprising a protector plate 600, a spacer 500, and a standard liner 100 fitted together. In this preferred embodiment, the spacer 500 is a high size spacer allowing the liner 100 to be at a more elevated position. By raising the liner 100 higher, the height of the joint is increased allowing for a tight and stable final implant. In this preferred embodiment, since the liner 100 is a standard liner, the angular movement of the liner 100 or of the spacer 500 does not affect the angle of orientation of the liner 100.

In a first step of a preferred method, the user creates a resection of the top of a bone according to the normal standards in the state of the art. Subsequently in a second step, the user uses a plastic or metal template which is placed on the recessed head resected surface to assess the size of the protector plate required. Once the optimal place is reached, the operator presses the template into the cancellous bone. The pins allow the template to remain secure on top of the resected surface. Using a drill bit or a reamer, a pre-marked and defined short hole is drilled via the through-hole into the spongy bone. Subsequently, the template is removed and a protector plate of the right size as described above is pressed or reamed in its place in the pre-marked pin and drilled holes.

Following the marking steps above, the user may use a protector plate 600, a spacer 500, and a corrective liner 300 fitted together to define the actual trial as shown in Figure 9 which would be equivalent to the final implant. In Figure 9, the user chooses a spacer 500 which is a low size spacer allowing the liner 300 to be at a lower position. By lowering the liner 300 through a shallower spacer 500 this allows the operator more clearance in the joint when this is too tight. In this example, since the liner is a corrective liner 300, the angular movement of the liner 300 or of the spacer 500 has a large effect upon the angle orientation of the final implant. Depending on the angle of orientation, the liner 300 may close or open the gap to the opposite counterpart bone joint. In this method, the operator may therefore try out a multitude of angular and height positions in very precise and small steps. The advantage of the present trials is that these method steps can be easily done without entirely extracting the liner 300 from the spacer 500 and/or the protector plate 600. The angular orientation may be in steps of any of 1° to 10°. In this preferred method, the user anchored the liner 300 to the spacer 500 by flexible spread feet 310. The spread feet ridges 320 fit in the spacer inner ridges 545 of the spacer 500 wherein the spread feet 510 of the spacer 500 fit in the inner ridges 610 of the protector 600 plate. All these parts are maintained in place due to the pressure force exerted upon the flexible spread feet. This assembly of elements and the method is best shown in figure 7.

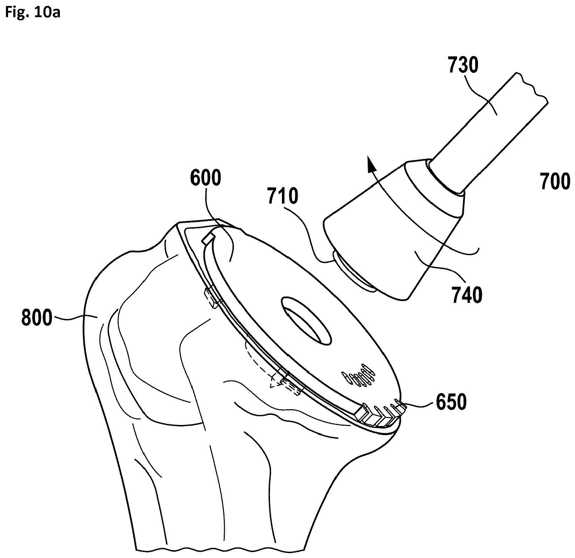

In a further step, once the height and orientation of the liner 100, 200, 300, 400 have been successfully determined, the system can be disassembled. The liner 100, 200, 300, 400 is carefully extracted by gripping it from the lateral finger recesses 180, 280, 380, 480 and lifting it from the spacer 500 or the protector plate 600. The spacer is then extracted from the protector. Finally, the protector plate 600 is also carefully extracted from the bone 800 with the help of the removal tool 700 as is shown in figure 10a.

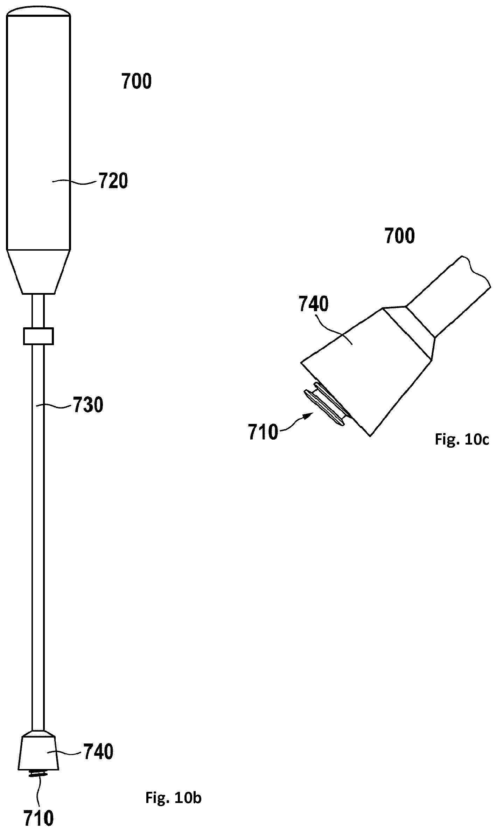

The removal tool 700 as can be seen in figures 10b and 10c has a handle 710 and a removal shaft 720 which has at its distal end a removal screw end 730 which is screwed with the help of a screw end thread 740 into the central spacer inner hole snap connector 540 of the spacer 500. When there is no spacer the removal tool 700 is attached to the protector plate inner hole 610 of the protector plate 600. Subsequently, the spacer 500 or the protector plate 600 may be lifted from the top of the bone by pulling the removal tool handle 710. In another method, the liner may be placed directly on the protector without the use of the spacer. The steps are basically the same as described above, but without the spacer 500.

### List of reference numerals

- 100: standard liner
- 105: central liner snap connector
- 110: spread feet
- 120: spread feet ridges
- 130: lateral fixing nipple
- 140: cup wall
- 150: glenosphere cup
- 151: top side
- 152: bottom side
- 161: lateral liner grasping channels
- 170: spread feet base
- 180: lateral finger recesses
- 190: angle direction line
- 195: rectangular liner rotation lock

- 200: standard constrained liner
- 205: central liner snap connector
- 210: spread feet
- 220: spread feet ridges
- 230: lateral fixing nipple
- 240: cup wall
- 250: glenosphere cup
- 251: top side
- 252: bottom side
- 260: constrained wall
- 261: lateral liner grasping channels
- 270: spread feet base
- 280: lateral finger recesses
- 290: angle direction line
- 295: rectangular liner rotation lock

- 300: corrective liner
- 305: central liner snap connector
- 310: spread feet
- 320: spread feet ridges
- 330: lateral fixing nipple
- 340: cup wall
- 350: glenosphere cup
- 351: top side
- 352: bottom side
- 360: corrective wall
- 361: lateral liner grasping channels
- 370: spread feet base
- 380: lateral finger recesses
- 390: angle direction line
- 395: rectangular liner rotation lock

- 400: corrective constrained liner
- 405: central liner snap connector
- 410: spread feet
- 420: spread feet ridges
- 430: lateral fixing nipple
- 440: cup wall
- 450: glenosphere cup
- 451: top side
- 452: bottom side
- 460: corrective constrain wall
- 461: lateral liner grasping channels

- 470: spread feet base
- 480: lateral finger recesses
- 490: angle direction line
- 495: rectangular liner rotation lock

- 500: spacer
- 505: central spacer snap connector
- 510: spread feet
- 520: spread feet ridges
- 530: lateral finger ridges
- 540: central spacer inner hole snap connector
- 545: spacer inner ridge
- 550: innertube
- 551: top side
- 552: bottom side
- 560: spacer nipple
- 561: lateral spacer grasping channels
- 570: spread feet base
- 575: rectangular rotation spacer lock space
- 580: spacer fixing nipple hole
- 585: central spacer snap connector transverse channel
- 590: angle indication line595 rectangular liner rotation lock
- 600: protector plate

- 610: protector plate inner hole
- 615: protector plate inner ridges
- 620: central post
- 630: lateral positioning teeth
- 640: angular holes
- 650: angular markings
- 651: top section
- 652: bottom section
- 660: anchoring pins
- 661: lateral protector plate grasping channels
- 675: rectangular rotation protector lock space
- 700: removal tool
- 710: removal tool handle
- 720: removal shaft
- 730: removal screw end
- 740: screw end thread

- 800: bone

## Claims

1. A trial implant system comprising a liner (100, 200, 300, 400), a spacer (500), and a protector plate (600),
the liner (100, 200, 300, 400) having a top side (151, 251, 351, 451) and a bottom side (152, 252, 352, 452), the liner (100, 200, 300, 400) further comprising:
- a glenosphere cup (150, 250, 350, 450) on the top side, and
- a central liner snap connector (105, 205, 305, 405) at the bottom side, and
the spacer having a top side (551) and a bottom side (552), the spacer further comprising:
- a central spacer inner hole snap connector (540) at the top side (551), and
- a central spacer snap connector (505) at the bottom side (552), and the protector plate (600) having a top section (651) and a bottom section (652), the protector plate (600) further comprising:
- a central post (620) extending centrally from the top section (651) through the bottom section (652) of the protector plate (600), and
the central post (620) further comprising a central protector plate inner hole (610),
the central liner snap connector (105, 205, 305, 405) fitting into the central spacer inner hole snap connector (540), and
the central spacer snap connector (505) fitting through the protector plate inner hole (610)).

2. A trial implant system comprising a liner (100, 200, 300, 400) and a protector plate (600), wherein
the liner (100, 200, 300, 400) has a top side (151, 251, 351, 451) and a bottom side (152, 252, 352, 452), the liner (100, 200, 300, 400) further comprising:
- a glenosphere cup (150, 250, 350, 450) on the top side (151, 251, 351, 451), and
- a central liner snap connector (105, 205, 305, 405) at the bottom side (152, 252, 352, 452), and
the protector plate (600) has a top section (651) and a bottom section (652), the protector plate further comprising:
- a central post (620) extending centrally from the top section (651) through the bottom section (652) of the protector plate (600), and
at least one anchoring pin (660), extending from the bottom section the central post (620) further comprises a central protector plate inner hole (610) and
the central liner snap connector (105, 205, 305, 405) fits through the protector plate inner hole (610).

3. The trial implant system according to claim 1 or 2,
**characterized in that**
the central liner snap connector (105, 205, 305, 405) has a spread feet base (170, 270, 370, 470) at the bottom side (152, 252, 352, 452) and flexible spread feet (110, 210, 310, 410) extending centrally and distally from the spread feet base (170, 270, 370, 470) and/or at least one anchoring pin (660), extending from the bottom section (652) of the protector plate (600) and/or at least one lateral positioning tooth (630) extending from the bottom section (652) of the protector plate (600).

4. The trial implant system according to any one of the preceding claims,
**characterized in that**
the liner (100, 200, 300, 400) has a fixing lateral nipple (130, 230, 330, 430) placed laterally from said spread feet (110, 210, 310, 410).

5. The trial implant system according to any one of claims 1, 3 and 4,
**characterized in that**
the central spacer inner hole snap connector (540) has inner ridges (545).

6. The trial implant system according to claim 5,
**characterized in that**
the protector plate inner hole (610) has protector plate inner ridges (615).

7. The trial implant system according to any one of claims 5 or 6,
**characterized in that**
said liner spread feet (110, 210, 310, 410) comprise exterior ridges (120, 220, 320, 420).

8. The trial implant system according to claim 7,
**characterized in that**
said liner spread feet (110, 210, 310, 410) comprises exterior ridges (120, 220, 320, 420) to match the inner ridges (545) of the spacer (500) or the inner ridge (615) of the central post (620) of the protector (600) allowing liners (100, 200, 300, 400) to be held in place once inserted and fitted in the spacer (500) and/or protector (600).

9. The trial implant system according to claim 8,
**characterized in that**
the exterior ridges (120) of the flexible spread feet (110, 210, 310, 410) are spaced at regular intervals of 1mm, 2mm or 3mm from each other.

10. The trial implant system according to any one of the preceding claims,
**characterized in that**
the liner cup wall (140, 240, 340, 440) has opposing lateral finger recesses (180, 280, 380, 480) extending in an archway from the bottom side (152, 252, 352, 452) towards the top side (151, 251, 351, 451).

11. The trial implant system according to any one of the preceding claims,
**characterized in that**
the protector module (600) has lateral angular holes (640) for allowing angle alignment of the liners (100, 200, 300, 400) and the spacer.

12. The trial implant system according to any one of the preceding claims,
**characterized in that**
the liner (100, 200, 300, 400) having lateral fixing nipples (140, 240, 340, 440) to penetrate the spacer (500) through the spacer fixing nipple hole (580) or to penetrate the protector (600) through the angular holes (640).

13. The trial implant system according to any one of the preceding claims,
**characterized in that**
the protector, the spacer, and the liner have different radial sizes from 20-50 mm and more preferably from 27 - 42 mm.

14. The trial implant system according to any one of the preceding claims,
**characterized in that**
the spacer (500) may vary in height from 3mm to 6mm.

15. The trial implant system according to any one of the preceding claims,
**characterized in that**
the liner (100) the spacer module (500) and the protector module (600) are made of plastic of different strengths and/or different colors.
